# EUROPEAN PATENT SPECIFICATION

(11) **EP 2 282 840 B1**
(45) Date of publication and mention of the grant of the patent: **13.11.2013**
(21) Application number: 09735546.5
(22) Date of filing: 20.04.2009
(51) Int. Cl.: B05B 9/04, B05B 5/16, B05B 5/025, B05B 1/14, B05B 1/30, A61M 16/04, B05B 1/32, A61M 15/02

(54) **ELECTROSPRAYING DEVICE**
ELEKTROSPRÜHVORRICHTUNG
DISPOSITIF D ÉLECTROPULVÉRISATION

(30) Priority: 22.04.2008 IE 20080310
(43) Date of publication of application: 16.02.2011
(73) Proprietor: National University of Ireland, Maynooth, Maynooth, County Kildare (IE)
(72) Inventor: O'DEA, Shirley, Maynooth County Kildare (IE); MAGUIRE, Michael, Dublin 4 (IE)
(74) Representative: Brophy, David Timothy
(86) International application number: PCT/EP2009/054680
(87) International publication number: WO 2009/130187

(56) References cited:
- WO-A-2004/078244
- DE-A1- 10 129 250
- US-A1- 2003 205 631
- US-B1- 7 258 285
- US-B2- 7 152 817
- MAMANEE W ET AL: "PDMS Based Thermopnuematic Peristaltic Micropump for Microfluidic Systems" JOURNAL OF PHYSICS: CONFERENCE SERIES, INSTITUTE OF PHYSICS PUBLISHING, BRISTOL, GB, vol. 34, no. 1, 1 April 2006 (2006-04-01), pages 564-569, XP020109664 ISSN: 1742-6596

## Description

### Technical field

This invention relates to the field of electrospraying, in particular to improved electrospraying devices and methods.

### Background Art

Electrospraying employs electrostatic repulsion and acceleration resulting from a high voltage to create an aerosol or plume of droplets. Typically, the droplets will be a volatile solvent in which a molecule, ion or particle of interest is suspended or dissolved.

Evaporation of the solvent from the drop surface causes the space charge effects on an ever-decreasing droplet to make the droplet unstable, disintegrating into smaller, highly charged droplets, which can disintegrate entirely due to the high charge concentration.

A typical electrospray system will comprise, at its most basic:
- a liquid supply source,
- a capillary, needle, nozzle, or other delivery orifice leading from the liquid supply source,
- a high voltage electrical source for applying a strong electric field to the liquid emerging from the open end of the delivery tube, and
- a counter-electrode, which may be any surface to which the droplets are attracted (thus while the counter-electrode forms part of the electrospray system, it is often not an explicit part of the apparatus, since it may be an external surface towards which the nozzle approaches).

Electrospraying finds applications in various fields, including thin film deposition processes, coating and painting processes, and mass spectroscopy. US Patent Specification No. 6,764,720 discloses the use of electrospraying techniques for delivering drug particles into cells.

A consistent problem with electrospraying techniques is the reliable creation of an electrospray. The creation of a plume which will form an electrospray tends to be a matter of trial and error. This limits the use of the electrospray technique in any application where the liquid to be sprayed may frequently vary, since a change in the liquid properties (e.g. conductivity or viscosity) will generally mean that the parameters used to create an electrospray (source to target potential difference, liquid flow rate, nozzle dimensions) must also be varied.

The result is that one cannot easily pump a wide range of liquids through an electrospray apparatus and expect a reliable electrospray creation, since various parameters, equipment settings and even nozzles may need to be changed in order for a plume to result and an electrospray be formed.

US 2003/0205631 discloses a device for electrospraying a liquid which has a peristaltic pump to transfer the liquid from a reservoir to the electrospray nozzle. US 7,152,817 discloses a device which similarly uses a peristaltic pump both to deliver liquid from the reservoir to the spray head and to electrically isolate the high voltage spray head from the product reservoir.

### Disclosure of the Invention

The invention provides an electrospray head for use in an electrospraying apparatus, according to claim 1, and an electrospray device according to claim 14.

The electrospray head has:
a body;
an actuator disposed in said body;
an internal surface in said body defining a conduit, said conduit having an inlet for receiving a fluid to be sprayed and an outlet for emitting said fluid; and
a movable wall forming a portion of said internal surface defining the conduit;
wherein said movable wall is arranged to co-operate with said actuator to constrict or dilate said conduit upon operation of the actuator.

Conventional electrospray heads take the form of a capillary tube, needle, or other tube of fixed diameter, so the only control over the fluid dynamics in such systems for a given liquid is control over the pumping pressure applied.

By providing a movable wall which can constrict or dilate the conduit from which the liquid emerges out of the electrospray head, an additional element of control is provided to affect the fluid dynamics, and thereby one obtains an additional way of influencing the creation of the plume for an electrospray.

The movable wall is located adjacent the outlet of the conduit whereby the effective exit diameter of the conduit can be varied by controlling the actuator.

In preferred embodiments, the movable wall comprises a cylindrical wall section whose diameter can be varied by controlling the actuator.

Preferably, said cylindrical wall section extends around the entire circumference of a cylinder.

Alternatively, said cylindrical wall section may extend around a sector of the circumference of a cylinder.

Further, preferably, said actuator is disposed circumferentially outside said cylindrical wall section.

Most preferably, said actuator is a generally cylindrical actuator operable to compress said cylindrical wall section and thereby constrict the diameter thereof.

The actuator comprises an expandable member disposed adjacent said movable wall, and means for varying the expansion of said expandable member.

Suitably, said expandable member comprises a fluid reservoir which may be caused to expand.

Preferably, said movable wall comprises a boundary of said reservoir.

Preferably, said fluid reservoir is constrained from movement in at least one direction, whereby the expansion of the fluid reservoir is enhanced in a different direction.

More preferably, said body is a solid structure having a seat within which said fluid reservoir is disposed.

Most preferably, said fluid reservoir is in the form of an annular cylinder having an inner cylindrical face defining said movable wall and an external cylindrical face abutting said seat in which said annular cylindrical reservoir is seated, whereby radial expansion of the reservoir is directed inwardly to constrict said inner cylindrical diameter.

Preferably, the expandable member is a thermally expandable structure, and the means for varying the expansion thereof comprises means for heating the thermally expandable structure.

Suitably, the means for heating comprises at least one resistor.

Preferably, when the fluid reservoir is in the form of an annular cylinder, the means for heating comprises a plurality of resistors disposed radially outwardly of the annular cylindrical reservoir.

In a preferred construction, the plurality of resistors are embedded in the body and flush with the seat.

The movable wall may be controllable to simultaneously constrict and dilate different portions of said conduit.

Preferably, in such cases, the movable wall is controllable to generate a peristaltic contraction movable along said conduit.

Further, preferably, said movable wall comprises a plurality of independently controllable sections which together define said conduit, and each independently controllable section being arranged to co-operate with an actuator to constrict or dilate a portion of said conduit upon operation of the actuator.

Optionally, the outlet of said conduit comprises a plurality of outlet orifices.

This plurality of outlet orifices may be configured to direct fluid emerging therefrom in a plurality of different directions.

Optionally, the electrospraying head further includes an electrode adapted to energise said fluid with a high voltage when connected to an appropriate power supply.

Suitably, the head may further include an electrical circuit for supplying said electrode with a high voltage.

Alternatively the electrode may be supplied from an external circuit

In a preferred embodiment, said body comprises a solid body structure having fabricated therein: said conduit; a space for receiving said movable wall; and a space for receiving said actuator.

Preferably, said solid body structure is selected from a substrate of borosilicate, quartz, or silicon.

The invention also provides an electrospraying device comprising an electrospray head as disclosed above, and means for receiving a supply of fluid for electrospraying.

A preferred implementation is a bronchoscope apparatus comprising such an electrospraying device mounted on a body adapted for insertion into the respiratory passages of a subject, whereby the electrospray head of the device may be directed at a tissue wall for delivery of an agent to said tissue by electrospraying.

Another preferred implementation is an apparatus for delivering a biological agent to a sample of cells *in vitro,* comprising a sample area for receiving said sample of cells, and an electrospraying device as aforesaid, the electrospraying head of said electrospraying device being positioned to deliver said biological agent from said supply of fluid to said sample of cells by electrospraying, and optionally, a counter-electrode positioned relative to the sample area to direct said electrospray.

The invention also provides a method of treatment of the human or animal body comprising the steps of providing an apparatus having an electrospray head as described herein, and operating said apparatus to deliver an electrospray agent to said human or animal body.

The invention will now be illustrated by the following description of embodiments thereof, given by way of example only with reference to the accompanying drawings, in which:
Fig. 1 is a perspective view of an electrospray head, shown from above;
Fig. 2 is a perspective view of the electrospray head of Fig. 1, shown from below;
Fig. 3 is a cross-sectional elevation of the electrospray head of Figs. 1 and 2;
Fig. 4 is a cross-sectional elevation of a second electrospray head;
Fig. 5 is a schematic diagram of an apparatus for electrospraying a liquid, in the form of a bronchoscope;
Fig. 6 shows a laboratory or industrial system for delivering an electrospray;
Fig. 7 is a cross-sectional elevation of a third electrospray head;
Fig. 8 is a cross-sectional elevation of a fourth electrospray head;
Fig. 9 is an end elevation view of the device of Fig. 8; and
Fig. 10 is a cross-sectional elevation of a fifth electrospray head.

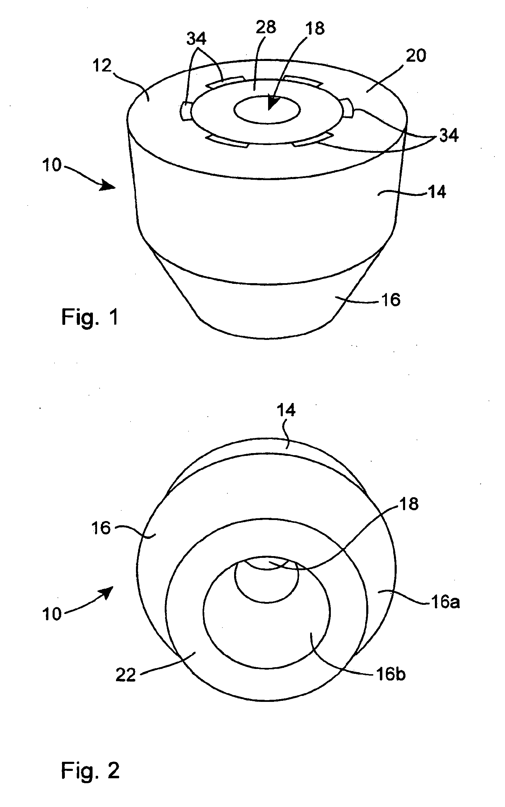

In Fig. 1 there is indicated, generally at 10, an electrospray head. The head has a body 12 of borosilicate glass, which has been fabricated to the desired shape, with a generally cylindrical upper section 14 and a tapering lower section 16. A central bore 18 is formed to provide a conduit for a liquid to be sprayed when supplied from a source (not shown) to which the electrospray head is connected, typically as part of an electrospray device adapted to receive the head.

Referring additionally to Fig. 2, which shows the head 10 from below, it can be seen that bore 18 extends from the upper surface 20 (Fig. 1) to the lower section 16 of the body, and that the lower section has an internal outwardly tapering surface 16b in addition to the external inwardly tapering surface 16a, these tapering faces being connected by an annular flat bottom face 22.

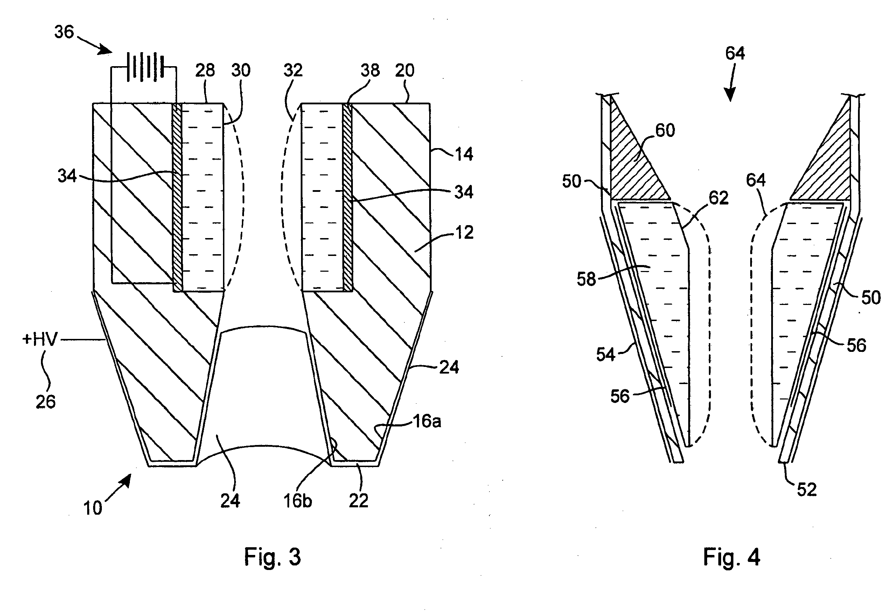

As shown in Fig. 3, a coating 24 of conductive material covers the major part of the tapering surfaces 16a,16b and annular bottom face 22, and this coating 24 provides a high voltage (HV) electrode when connected to an appropriate HV source 26.

As is generally understood in the art, and without wishing to be bound by any theory, liquid emerging from the bore 18 is exposed to an electric field, and the shape of liquid starts to deform from the shape caused by surface tension alone. Under a high voltage, the effect of the electric field becomes more prominent until a cone shape begins to form with convex sides and a rounded tip (a Taylor cone). When a certain threshold voltage has been reached the slightly rounded tip inverts and emits a jet of liquid. This is called a cone-jet and is the beginning of the electrospray process.

The formation of the cone, and thus the electrospray, is influenced by several factors, including the viscosity and conductivity of the liquid, the applied voltage, the geometry of the electrode and of the electric field, the diameter and shape of the outlet from the bore from which the liquid emerges, the flow rate of the liquid, the humidity and constitution of the surrounding gas medium, the electric potential, geometry, and distance to the nearest counter-electrode surface, and so on.

The electrospray head of Figs. 1-3 includes an annular cylindrical reservoir 28 whose inner wall 30 defines the surface of the conduit or bore 18 through which liquid flows. The reservoir is formed of a silicone body filled with a liquid having a coefficient of thermal expansion suitable to expand the flexible inner wall 30 (as shown by dotted line 32) to a sufficient degree to control the exit diameter from the conduit, and also the flow rate through the conduit.

An example of a suitable liquid is one of the family of perfluorinated liquids sold by 3M under the trade mark Fluorinert. For example, the product sold as Fluorinert-70 has a coefficient of thermal expansion of 0.001 per degree centigrade (at 25 degrees centigrade) and is suitable for use as a thermally expanding fluid.

Other candidate fluids (with coefficient of thermal expansion indicated in parentheses) include:

| | |
|---|---|
| Gasoline | (0.000950 per degree centigrade) |
| Ethanol | (0.000750 per degree centigrade) |
| Water | (0.000207 per degree centigrade) |
| Mercury | (0.000182 per degree centigrade) |

The silicone material of the reservoir walls has a coefficient of thermal expansion of 0.0009 per degree centigrade, and is thus well matched with the Fluorinert liquid. (This also suggests that a solid silicon block could be employed, though this would suffer from a significant lag and from uneven heating - the Fluorinert, being used primarily as a coolant for electronics, promotes rapid equalization of temperature and thus more rapid response of the reservoir to applied temperature changes.

As an example, a suitable reservoir may have the following dimensions:
Inner diameter of reservoir (at 20 degrees C): 300 microns
Outer diameter of reservoir: 4.54mm
Height of cylinder: 10mm
Wall thickness at inside diameter: 100 microns

Thus, at 20 degrees centigrade, the inner diameter of this reservoir is 300 microns. When heat is applied, the volumetric expansion of the reservoir is confined from the exterior and from below by the solid body of borosilicate, and is confined from above by virtue of the top wall being thicker and more rigid than the inner wall 30. Accordingly, the inner diameter is constricted as the reservoir expands, and the following results are seen:

**Table 1: diameter of orifice as a function of temperature**

| **Orifice Diameter (microns)** | **Temperature (degrees C)** |
|---|---|
| 300 | 20.0 |
| 290 | 22.4 |
| 280 | 24.7 |
| 250 | 31.0 |
| 200 | 39.8 |
| 150 | 47.0 |
| 100 | 53.1 |
| 50 | 58.2 |

There are two important points to note from Table 1. The first is that the relationship is close to linear, so that extrapolation and calibration is easily achievable. The second point to note is that a large range of orifice diameters is achievable with only a modest increase in temperature.

Referring back to Fig. 3, the temperature is varied by means of a number of resistors 34 which are energised by a suitable power supply 36. As seen from Fig. 1, the resistors are distributed circumferentially around the outside of the reservoir 28 at equal intervals.

The resistors 34 are shown as having a significant thickness in Figs. 1 and 3, and are accommodated in a series of cavities which are fabricated into the surface of the cylindrical seating 38 which seats the reservoir 28. In practice, the resistors may be deposited as thin film resistors on the surface 38 of the cylindrical seating before the reservoir has been inserted.

Typically, a thermometer (not shown) such as a platinum resistance thermometer, for example, will be provided in the reservoir or in contact with the reservoir to monitor the temperature (and by extrapolation, indicate the orifice diameter. Suitable control circuitry is used to adjust the power supply to the resistors to maintain the reservoir at the correct temperature for the desired orifice diameter.

More sophisticated control circuitry can be provided which is programmable to apply the correct temperature (or temperature cycle) for a given liquid to be sprayed. Thus, if it is desired to spray a variety of liquids through an electrospraying device, the electrospray head of Figs. 1-3 may provide the user with the ability to employ a single electrospray head with a controllable orifice output diameter, where conventional electrospraying devices would have required changing the head or capillary tube for the different liquids to be successfully sprayed.

Fig. 4 shows an alternative electrospray head, having a hollow body 50 tapering towards an outlet tip 52. The body may be made of a dielectric material such as glass or plastic. A conductive coating 54 on the exterior surface of the tapering body provides an electrode for application of a high voltage electric field, to create an electrospray of liquid emerging from the tip 52.

On the interior of the tapering body, a plurality of thin film resistors 56 have been deposited at intervals around the circumference of the inner surface, to provide a means for controllably applying heat when energised by a suitable power source and control circuitry (not shown).

Inwardly of the resistors, a reservoir 58 having silicone walls and being filled with Fluorinert-70, is held in place by a bulkhead 60 and by the shape of the tapering tip. The inner wall 62 of the reservoir 58 thus forms a part of a conduit leading from an inlet to the body (not shown, but liquid coming from the inlet travels in the direction indicated by arrow 64), to the outlet tip 52.

Thus, when the resistors 56 are heated, the reservoir undergoes thermal expansion in the only available direction, i.e. inwardly. This causes the inner wall 62 to bulge inwardly, as indicated by the dotted lines 66, thereby constricting the conduit at a point adjacent to the outlet thereof.

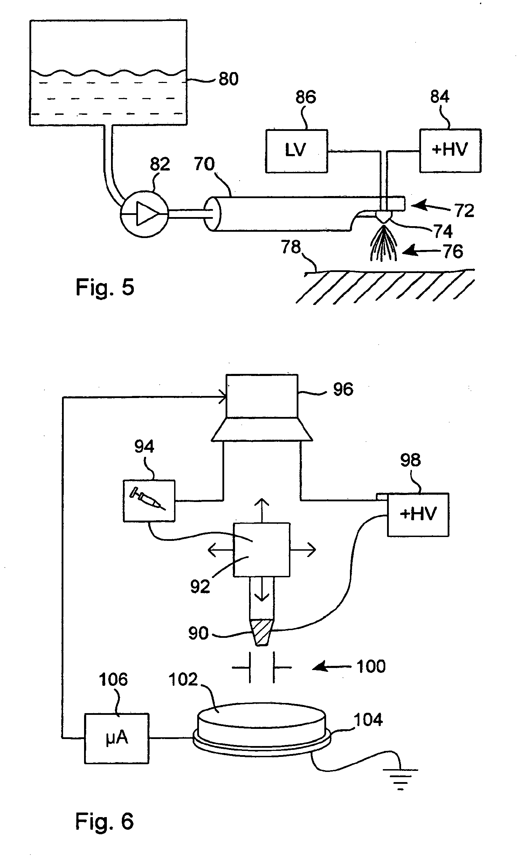

Fig. 5 shows a schematic diagram of an apparatus for electrospraying a liquid, in the form of a bronchoscope 70, the form and function of which is generally known. Bronchoscopes allow the passage of instruments into the bronchial passages under the control of a surgeon or operative, and are controllable by manipulative controls (not shown) under the guidance of cameras typically provided towards the distal end 72 of the scope.

The bronchoscope is equipped with an electrospray head 74 as described herein. The electrospray head is directed laterally so that a spray 76 may be directed at a wall 78 of the bronchial passages.

The bronchoscope is supplied with a liquid supply from a reservoir 80 and pump 82, whereby liquid can be supplied at an appropriate flow rate to the electrospray head 74. The liquid will preferably contain an active ingredient, and particularly of interest are liquids including but not limited to:

### Therapeutic biological molecules

- DNA-based
- RNA-based
- protein-based (including, but not limited to: small peptides, proteins, antibodies, growth factors, vaccines)
- lipid-based
- carbohydrate-based

### Therapeutic non-biological molecules

- chemical molecules
- radio-isotopes
- chemotherapeutic molecules

### Diagnostic molecules

- labelled/detectable molecules (including, but not limited to: radio-labels)
- metal particles
which can be accelerated into the cells of the tissue wall 78 to thereby transfect the cells.

Control of the electrospray is effected not by a variable high voltage supply 84 and by the low voltage controllable power supply 86 which feeds the heating resistors of the electrospray head.

Additional control means may be included (not shown) including in particular motors to direct the electrospray head, and directional electrodes to deflect, disperse or concentrate the charged electrospray as desired.

Fig. 6 shows a laboratory or industrial system for delivering an electrospray *in vitro.* Again this system is shown in highly schematic form for clarity.

An electrospray head 90 is mounted on a micromanipulator 92 which controls the precise position of the head 90 along the X-, Y- and Z-axes. Liquid is supplied to the conduit in the head 90 from a syringe driver 94 under the control of a computer 96.

In another embodiment the liquid may be present in a reservoir close to the head 90 and the liquid flow to the head supplied via an peristaltic action pump between the reservoir and the head.

High voltage power is supplied from a HV power supply 98 to the HV electrode on the electrospray head, the HV power supply similarly being controlled by the computer 96. The low voltage power supply feeding the heating resistors is omitted for clarity, but again the temperature is controllable by the computer, which may be programmed with parameters for spraying different liquids, or which may operate in a learning mode to empirically determine the optimum parameters for a given liquid to be sprayed.

Directional electrodes 100 are positioned to control the direction and shape of the electrospray emerging from the head 90, with this spray being directed to a sample area 102 mounted over a counter-electrode 104. The sample area may be an array of sample cells, each containing an isolated sample, such that the micromanipulator and directional electrodes are operable to control the delivery of liquid to individual sample cells in the array.

A micro-ammeter 106 or other charge/current detector is provided to enable the computer control program to monitor the quantity of charge delivered to the sample area, and thus by inference, the dosage of liquid being delivered over time to individual areas of the sample array where there is a monotonic relationship between the charge collected at the target and the liquid dosage delivered to the target.

Using this apparatus, it is possible to transfect biological samples with DNA or with other biologically active agents in a controlled manner.

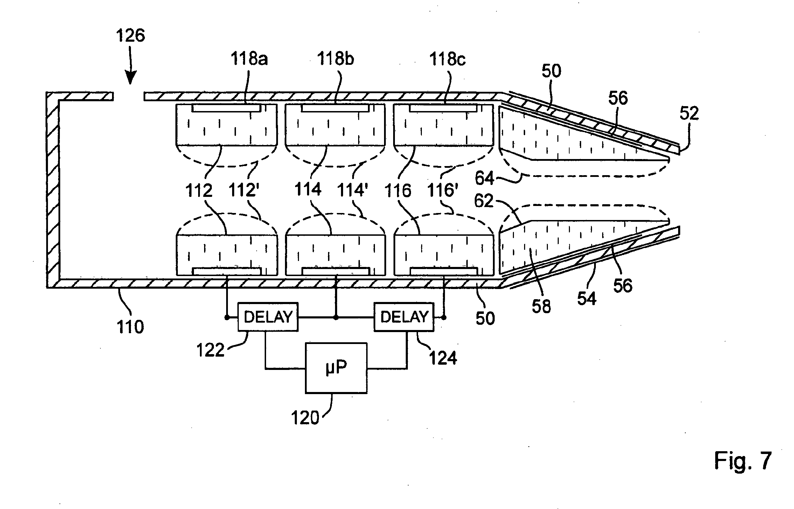

Fig. 7 shows an electrospray device which is based on the spray head of Fig. 4. Like parts are designated with like reference numerals, and the spray tip operates just as previously described.

The device of Fig. 7 has a delivery barrel 110 which is continuous with the hollow body portion 50 leading to the spray head. A plurality of cylindrical peristalsis reservoirs 112,114,116 are arranged in series along the interior of the barrel 110. These peristalsis reservoirs are each provided with a respective annular heating electrode 118a, 118b, 118c so that they can be controllably heated and thereby expanded to constrict the internal volume defined by the respective cylinders as shown in dotted outline at 112', 114', 116'.

Control of the heating electrodes 118a, 118b, 118c is carried out by a microprocessor 120 which is programmed to implement a delay function (indicated schematically as 122,124 between the heating of each successive electrode 118a, 118b, 118c in series. In this way a peristaltic action is generated along the interior volume defined within the cylindrical peristaltic reservoirs 112,114,116, to assist in pumping fluid from a supply reservoir (not shown) as indicated by arrow 126.

It will be appreciated that in reality the number and arrangement of peristaltic reservoirs can be varied and in practice a greater number than three such reservoirs will be used. If the peristaltic action is sufficiently powerful, having regard to the required delivery rate of fluid through the electrospray head, the viscosity of the fluid, and so on, then a pressurised fluid supply may be dispensed with and the peristaltic action alone can be used to control the rate of delivery of fluid to the spray head.

It will also be appreciated that the reservoir 62 at the spray head itself can be operated as part of the chain of peristaltic reservoirs or can be operated independently to choke the fluid supply and thereby control delivery rate.

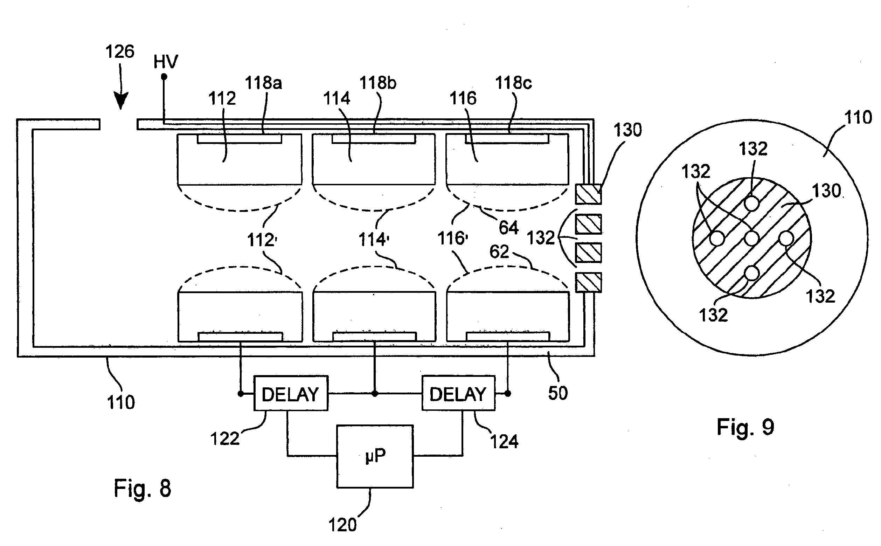

Fig. 8 shows a further alternative embodiment which is similar to that of Fig. 7 in terms of having a plurality of peristalsis reservoirs for pumping or controlling the flow of fluid from a supply 126 through a barrel 110. The same reference numerals have been used to designate those parts having the same function as in Fig. 7.

The spray head 130 of Fig. 8 is different in that it comprises multiple orifices 132 each spraying the fluid to be delivered in parallel directions. Such a spray head can readily be fabricated using MEMS techniques.

Fig. 9 shows the device of Fig. 8 in end elevation looking towards the spray head.

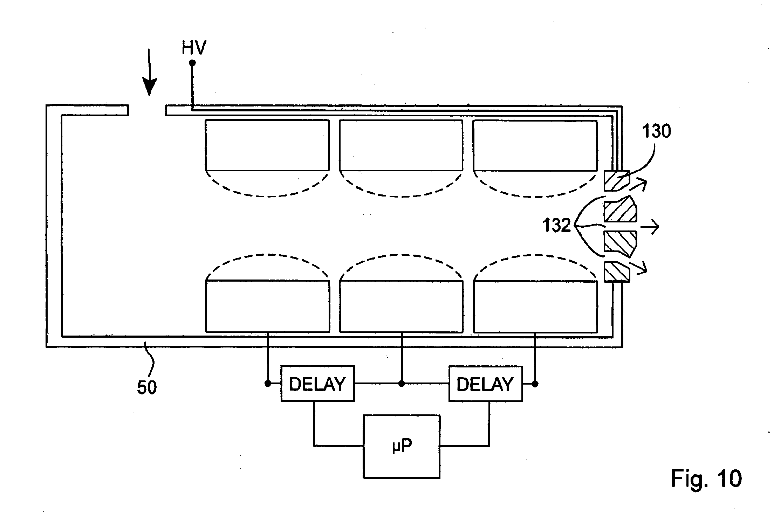

Fig. 10 shows a device which is identical to that of Figs. 8 and 9 except that the multiple orifices 132 are not parallel but instead diverge from one another to spray the fluid in a divergent spray formation from the spray head.

It will be appreciated that spray heads having multiple orifices such as the spray heads 130 of Figs. 8-10, need not be used with the peristaltic pumping system shown in Figs. 8 and 10, and such spray heads can equally be incorporated in devices which do not have such a peristaltic mechanism (such as the device of Fig. 1).

It will also be understood that the five orifices shown in Figs. 8-10 are illustrative only and the actual number may be any number from a single orifice upwards.

The invention is not limited to the embodiments herein which may be modified or varied without departing from the scope of the claimed invention.

## Claims

1. An electrospray head (10; 74; 90) for use in an electrospraying apparatus (70) the electrospray head comprising:
a body (12; 50; 110);
an actuator (28, 34, 36; 56, 58; 56, 112, 114, 116, 118a-c, 120, 124) disposed in said body;
an internal surface in said body defining a conduit (18), said conduit having an inlet (126) for receiving a fluid to be sprayed when supplied from a fluid source (80, 82; 94) to which the electrospray head is connected and an outlet (16b; 52; 132) for emitting said fluid;
**characterised by**:
a movable wall (30; 62; 112, 114, 116) located adjacent the outlet of the conduit, said movable wall forming a portion of said internal surface defining the conduit;
wherein said movable wall is arranged to co-operate with said actuator to constrict (32; 64; 112', 114' 116') or dilate (30; 62; 112, 114, 116) said conduit upon operation of the actuator, wherein the actuator comprises an expandable member (28; 58) disposed adjacent said movable wall, and means (34, 36; 56; 118a-c, 120, 122, 124) for varying the expansion of said expandable member;
whereby the effective exit dimensions of the conduit can be varied by controlling the actuator to provide an additional element of control to affect the fluid dynamics and the creation of an electrospray plume.

2. An electrospray head according to claim 1, wherein the movable wall comprises a cylindrical wall section (30; 62; 62, 112, 114, 116) whose diameter can be varied by controlling the actuator.

3. An electrospray head according to claim 2, wherein said cylindrical wall section extends around the entire circumference of a cylinder.

4. An electrospray head according to claim 3, wherein said actuator is a generally cylindrical actuator disposed outside said cylindrical wall section and operable to compress said cylindrical wall section and thereby constrict the diameter thereof.

5. An electrospray head as claimed in claim 1, wherein said expandable member comprises a fluid reservoir (28; 58) which may be caused to expand.

6. An electrospray head as claimed in claim 5, wherein said movable wall comprises a boundary (30; 62) of said reservoir.

7. An electrospray head as claimed in claim 5, wherein said body is a solid structure having a seat (38; 50, 60) within which said fluid reservoir is disposed.

8. An electrospray head as claimed in claim 7, wherein said fluid reservoir is in the form of an annular cylinder having an inner cylindrical face defining said movable wall and an external cylindrical face abutting said seat in which said annular cylindrical reservoir is seated, whereby radial expansion of the reservoir is directed inwardly to constrict said inner cylindrical diameter.

9. An electrospray head as claimed in any one of claims 4-8, wherein the expandable member is a thermally expandable structure, and wherein the means for varying the expansion thereof comprises means (34, 36; 56; 118a-c, 120, 122, 124) for heating the thermally expandable structure.

10. An electrospray head as claimed in claim 9, wherein the means for heating comprises at least one resistor (34; 56; 118a-c).

11. An electrospray head as claimed in claim 10, when dependent on claim 8, wherein the means for heating comprises a plurality of resistors (34; 56) disposed radially outwardly of the annular cylindrical reservoir.

12. An electrospray head as claimed in any preceding claim, wherein said movable wall (62, 112, 114, 116) is controllable to simultaneously constrict and dilate different portions of said conduit.

13. An electrospray head as claimed in any preceding claim, wherein said outlet of said conduit comprises a plurality of outlet orifices (132).

14. An electrospraying device (70) comprising an electrospray head (74) as claimed in any preceding claim, and means (70) for receiving a supply (80, 82) of fluid for electrospraying.

## Patentansprüche

1. Elektrosprühkopf (10; 74; 90) zur Verwendung in einer Elektrosprühvorrichtung (70), wobei der Elektrosprühkopf Folgendes umfasst:
einen Körper (12; 50; 110);
eine Betätigungsglied (28, 34, 36; 56, 58; 56, 112, 114, 116, 118a-c, 120, 124), das in dem Körper angeordnet ist;
eine innenliegende Oberfläche in dem Körper, die eine Leitung (18) definiert, wobei die Leitung einen Einlass (126) zum Empfangen eines zu sprühenden Fluids, wenn es von einer Fluidquelle (80, 82; 94), mit der der Elektrosprühkopf verbunden ist, zugeführt wird, und einen Auslass (16b; 52; 132) zum Ausstoßen des Fluids aufweist;
**gekennzeichnet durch**:
eine bewegliche Wand (30; 62; 112, 114, 116), die sich benachbart dem Auslass der Leitung befindet, wobei die bewegliche Wand einen Abschnitt der die Leitung definierenden innenliegenden Oberfläche bildet;
wobei die bewegliche Wand dazu angeordnet ist, mit dem Betätigungsglied zusammenzuwirken, um die Leitung bei Betätigung des Betätigungsglieds zu verengen (32; 64; 112', 114' 116') oder aufzuweiten (30; 62; 112, 114, 116), wobei das Betätigungsglied ein benachbart der beweglichen Wand angeordnetes ausdehnbares Glied (28; 58) und Mittel (34, 36; 56; 118a-c, 120, 122, 124) zum Verändern der Ausdehnung des ausdehnbaren Glieds umfasst;
wodurch die effektiven Austrittsabmessungen der Leitung verändert werden können, indem das Betätigungsglied dazu gesteuert wird, ein zusätzliches Element der Steuerung vorzusehen, um die Fluiddynamik und die Erzeugung einer Elektrosprühfahne zu beeinflussen.

2. Elektrosprühkopf nach Anspruch 1, wobei die bewegliche Wand einen zylindrischen Wandabschnitt (30; 62; 62, 112, 114, 116) umfasst, dessen Durchmesser durch Steuern des Betätigungsglieds verändert werden kann.

3. Elektrosprühkopf nach Anspruch 2, wobei sich der zylindrische Wandabschnitt um den gesamten Umfang eines Zylinders herum erstreckt.

4. Elektrosprühkopf nach Anspruch 3, wobei es sich bei dem Betätigungsglied um ein allgemein zylindrisches Betätigungsglied handelt, das außerhalb des zylindrischen Wandabschnitts angeordnet ist und wirksam ist, um den zylindrischen Wandabschnitt zusammenzudrücken und dadurch dessen Durchmesser einzuengen.

5. Elektrosprühkopf nach Anspruch 1, wobei das ausdehnbare Glied einen Fluidbehälter (28; 58) umfasst, der veranlasst werden kann, sich auszudehnen.

6. Elektrosprühkopf nach Anspruch 5, wobei die bewegliche Wand eine Grenze (30; 62) des Behälters umfasst.

7. Elektrosprühkopf nach Anspruch 5, wobei es sich bei dem Körper um eine massive Struktur mit einer Aufnahme (38; 50, 60) handelt, in der der Fluidbehälter angeordnet ist.

8. Elektrosprühkopf nach Anspruch 7, wobei der Fluidbehälter die Form eines ringförmigen Zylinders hat und eine die bewegliche Wand definierende innere zylindrische Oberfläche und eine an die Aufnahme, in der der ringförmige zylindrische Behälter aufgenommen ist, anstoßende äußere zylindrische Oberfläche aufweist, wodurch die radiale Ausdehnung des Behälters nach innen gerichtet ist, um den inneren zylindrischen Durchmesser einzuengen.

9. Elektrosprühkopf nach einem der Ansprüche 4-8, wobei es sich bei dem ausdehnbaren Glied um eine thermisch ausdehnbare Struktur handelt und wobei das Mittel zum Verändern der Ausdehnung desselben Mittel (34, 36; 56; 118a-c, 120, 122, 124) zum Erwärmen der thermisch ausdehnbaren Struktur umfasst.

10. Elektrosprühkopf nach Anspruch 9, wobei das Mittel zum Erwärmen mindestens einen Widerstand (34; 56; 118a-c) umfasst.

11. Elektrosprühkopf nach Anspruch 10, wenn abhängig von Anspruch 8, wobei das Mittel zum Erwärmen mehrere Widerstände (34; 56) umfasst, die radial außerhalb von dem ringförmigen zylindrischen Behälter angeordnet sind.

12. Elektrosprühkopf nach einem der vorangehenden Ansprüche, wobei die bewegliche Wand (62, 112, 114, 116) steuerbar ist, um gleichzeitig verschiedene Abschnitte der Leitung einzuengen und aufzuweiten.

13. Elektrosprühkopf nach einem der vorangehenden Ansprüche, wobei der Auslass der Leitung mehrere Auslassöffnungen (132) umfasst.

14. Elektrosprühvorrichtung (70), umfassend einen Elektrosprühkopf (74) nach einem der vorangehenden Ansprüche und Mittel (70) zum Aufnehmen eines Vorrats (80, 82) an Fluid zum Elektrosprühen.

## Revendications

1. Tête d'électrospray (10; 74 ; 90) destinée à être utilisée dans un appareil d'électrospray (70), la tête d'électrospray comprenant :
un corps (12; 50; 110);
un actionneur (28, 34, 36 ; 56, 58 ; 56, 112, 114, 116, 118a-c, 120, 124) disposé dans ledit corps ;
une surface interne dans ledit corps définissant un conduit (18), ledit conduit ayant une admission (126) pour recevoir un fluide à pulvériser lorsqu'il est fourni depuis une source de fluide (80, 82 ; 94) à laquelle la tête d'électrospray est connectée et une sortie (16b ; 52 ; 132) pour émettre ledit fluide ;
**caractérisée par** :
une paroi mobile (30; 62 ; 112, 114, 116) située à proximité de la sortie du conduit, ladite paroi mobile formant une partie de ladite surface interne définissant le conduit;
dans laquelle ladite paroi mobile est agencée pour coopérer avec ledit actionneur afin de rétrécir (32; 64 ; 112', 114' 116') ou dilater (30; 62 ; 112, 114, 116) ledit conduit au fonctionnement dudit actionneur, dans laquelle ledit actionneur comprend un élément expansible (28 ; 58) disposé à proximité de ladite paroi mobile, et un moyen (34, 36 ; 56 ; 118a-c, 120, 122, 124) pour faire varier l'expansion dudit élément expansible ;
de telle sorte que les dimensions de sortie effectives du conduit puissent être modifiées en commandant l'actionneur afin de fournir un élément supplémentaire de commande en vue d'affecter la dynamique fluidique et la création d'un panache d'électrospray.

2. Tête d'électrospray selon la revendication 1, dans laquelle la paroi mobile comprend une section de paroi cylindrique (30 ; 62 ; 62, 112, 114, 116) dont le diamètre peut être modifié par commande de l'actionneur.

3. Tête d'électrospray selon la revendication 2, dans laquelle ladite section de paroi cylindrique s'étend autour de toute la circonférence d'un cylindre.

4. Tête d'électrospray selon la revendication 3, dans laquelle ledit actionneur est un actionneur généralement cylindrique disposé en dehors de ladite section de paroi cylindrique et exploitable pour compresser ladite section de paroi cylindrique et rétrécir ainsi son diamètre.

5. Tête d'électrospray selon la revendication 1, dans laquelle ledit élément expansible comprend un réservoir de fluide (28 ; 58) qui peut être amené à gonfler.

6. Tête d'électrospray selon la revendication 5, dans laquelle ladite paroi mobile compose une délimitation (30 ; 62) dudit réservoir.

7. Tête d'électrospray selon la revendication 5, dans laquelle ledit corps est une structure solide ayant un siège (38 ; 50, 60) dans lequel ledit réservoir de fluide est disposé.

8. Tête d'électrospray selon la revendication 7, dans laquelle ledit réservoir de fluide se présente sous la forme d'un cylindre annulaire ayant une face cylindrique interne définissant ladite paroi mobile et une face cylindrique externe butant contre ledit siège dans lequel repose ledit réservoir cylindrique annulaire, de telle sorte que l'expansion radiale du réservoir soit dirigée vers l'intérieur afin de rétrécir ledit diamètre cylindrique interne.

9. Tête d'électrospray selon l'une quelconque des revendications 4-8, dans laquelle l'élément expansible est une structure thermiquement expansible, et dans laquelle le moyen pour faire varier son expansion comprend un moyen (34, 36 ; 56 ; 118a-c, 120, 122, 124) pour chauffer la structure thermiquement expansible.

10. Tête d'électrospray selon la revendication 9, dans laquelle le moyen de chauffage comprend au moins une résistance (34 ; 56 ; 118a-c).

11. Tête d'électrospray selon la revendication 10, dépendante de la revendication 8, dans laquelle le moyen de chauffage comprend une pluralité de résistances (34 ; 56) disposées radialement vers l'extérieur du réservoir cylindrique annulaire.

12. Tête d'électrospray selon l'une quelconque des revendications précédentes, dans laquelle ladite paroi mobile (62, 112, 114, 116) est contrôlable pour rétrécir et dilater simultanément des parties différentes dudit conduit.

13. Tête d'électrospray selon l'une quelconque des revendications précédentes, dans laquelle ladite sortie dudit conduit comprend une pluralité d'orifices de sortie (132).

14. Dispositif d'électrospray (70) comprenant une tête d'électrospray (74) selon l'une quelconque des revendications précédentes, et moyen (70) pour recevoir une alimentation (80, 82) de fluide d'électrospray.
